# EUROPEAN PATENT SPECIFICATION

(11) **EP 4 311 521 B1**
(45) Date of publication and mention of the grant of the patent: **04.11.2026**
(21) Application number: 23186209.5
(22) Date of filing: 18.07.2023
(51) Int. Cl.: A61F 2/07, A61F 2/95, A61F 2/966, A61F 2/06

(54) **PROSTHESIS FOR TREATING SHORT OR NO NECK ANEURYSMS**
PROTHESE ZUR BEHANDLUNG VON KURZEN ODER KEINEN HALSANEURYSMEN
PROTHÈSE DE TRAITEMENT D'ANEVRISMES COURTS OU SANS COLLET

(30) Priority: 29.07.2022 US 202217876944
(43) Date of publication of application: 31.01.2024
(73) Proprietor: Medtronic Vascular, Inc., Santa Rosa, CA 95403 (US)
(72) Inventor: PERKINS, Keith, Santa Rosa, 95403 (US); TRICARICO, Rosamaria, Santa Rosa, 95403 (US); SETHNA, Sohrab, Santa Rosa, 95403 (US)
(74) Representative: Zimmermann & Partner Patentanwälte mbB

(56) References cited:
- US-A1- 2006 184 229
- US-A1- 2017 290 654
- US-A1- 2020 383 769

## Description

### FIELD OF THE INVENTION

The present invention relates to medical devices and more particularly, a device to treat short neck, hostile neck, or no neck aortic pathologies and provide a stable landing zone within unfavorable anatomy.

### BACKGROUND

An aneurysm is an excessive localized enlargement of an artery caused by a weakening of the artery wall. Aneurysms often occur in the aorta, brain, back of the knee, intestine, or spleen. An abdominal aortic aneurysm (AAA) is an enlarged area in the lower part of the aorta. A thoracic aortic aneurysm (TAA) is a weakened area in the upper part of the aorta. AAA and TAA aneurysms may be treated with stent graft prostheses, which are endovascular devices that include a stent or stents coupled to graft material. The stent(s) are delivered in a radially compressed configuration and are radially expanded, often by self-expansion, to engage the wall of the vessel having the aneurysm. The graft material defines a lumen for blood to flow through such that pressure on the aneurysm is not increased. Stent graft prostheses are conventionally primarily anchored to the vessel proximal to the aneurysm. The portion of the vessel to which the stent graft prosthesis is anchored may be referred to as the landing zone or the seal zone.

Depending on the location and size of AAA and TAA aneurysms, including the location of the aneurysm relative to branch vessels, AAA and TTA aneurysms may lack a proximal neck that is suitable for endovascular repair due to either a short seal/landing zone or a high degree of landing zone angulation. Unfavorable anatomy limits the percent of potential patients that can be treated using a purely endovascular approach. Solutions such as customized devices ordered to match a patient's specific anatomy typically require at least six to eight weeks to manufacture.

The present disclosure relates to an improved endovascular device for providing a stable landing zone for endovascular prostheses to be deployed within an aorta of a patient. More particularly, the present invention relates to an engineered landing zone that allows an endovascular prosthesis to be deployed within an aorta that lacks a proximal neck that is suitable for endovascular repair due to either a short seal/landing zone or a high degree of landing zone angulation.
US 2020/383769 A1 describes a luminal stent.

### BRIEF SUMMARY OF THE INVENTION

In a first example, a method (not claimed) is provided for creating an engineered landing zone includes delivering a landing zone prosthesis in a radially compressed configuration to a site of an aneurysm within a vessel, the landing zone prosthesis including a frame, an engineered landing zone, and graft material coupled at a first end to the frame and at a second end to the engineered landing zone, wherein the graft material includes a non-stented portion between the frame and the engineered landing zone. The method further includes radially expanding the frame at the site of the aneurysm, wherein with the frame radially expanded, the engineered landing zone remains in the radially compressed configuration longitudinally spaced from the frame, securing the frame to the vessel, longitudinally translating the engineered landing zone such that the engineered landing zone is at least partially disposed within the frame, and radially expanding the engineered landing zone.

In a second example, in the method of the first example, securing the frame to the vessel comprises adhesively securing the frame and/or the graft material attached to the frame to the vessel.

In a third example, in the method of the first example, securing the frame to the vessel comprises delivering endoanchors to within the frame, and deploying the endoanchors through the frame and into the vessel to secure the frame to the vessel.

In a fourth example, in the method of the third example, deploying the endoanchors further comprises deploying the endoanchors through the graft material.

In a fifth example, in the method of any of the first through fourth examples, the engineered landing zone comprises at least one stent and landing zone graft material coupled to the at least one stent, wherein radially expanding the engineered landing zone comprises radially expanding the at least one stent.

In a sixth example, in the method of the fifth example, the at least one stent is self-expanding, and the step of radially expanding the at least one stent comprises releases the stent from a constraint to allow the at least one stent to self-expand.

In a seventh example, in the method of the fifth example or the sixth example, the landing zone graft material is different than the graft material.

In an eighth example, in the method of the fifth example or the sixth example, the landing zone graft material is an extension of the graft material.

In a ninth example, in the method of any one of the first through the eighth examples, the frame is self-expanding, and radially expanding the frame comprises releasing the frame from a constraint to allow the frame to self-expand.

In a tenth example, a method (not claimed) for bypassing an aneurysm includes delivering a landing zone prosthesis in a radially compressed configuration to a site of an aneurysm in a main vessel, the landing zone prosthesis including a frame, an engineered landing zone, a graft material coupled at a first end to the frame and at a second end to the engineered landing zone, wherein the graft material includes a non-stented portion between the frame and the engineered landing zone. The method further includes radially expanding the frame at the site of the aneurysm, wherein with the frame radially expanded, the engineered landing zone remains in the radially compressed configuration longitudinally spaced from the frame, securing the frame to the vessel, longitudinally translating the engineered landing zone such that the engineered landing zone is at least partially disposed within the frame, radially expanding the engineered landing zone, delivering an endovascular prosthesis to the site such that a portion of the endovascular prosthesis is disposed within the engineered landing zone, and radially expanding the endovascular prosthesis such that the portion of the endovascular prosthesis disposed within the engineered landing zone engages the engineered landing zone.

In an eleventh example, in the method of the tenth example, securing the frame to the vessel comprises adhesively securing the frame and/or the graft material attached to the frame to the vessel.

In a twelfth example, in the method of the tenth example, securing the frame to the vessel comprises delivering endoanchors to within the frame, and deploying the endoanchors through the frame and into the vessel to secure the frame to the vessel.

In a thirteenth example, in the method of the twelfth example, the graft material lines at least a portion of the frame, and deploying the endoanchors further comprises deploying the endoanchors through the graft material.

In a fourteenth example, in the method of any one of the tenth through thirteenth examples, the engineered landing zone comprises at least one stent and landing zone graft material coupled to the at least one stent, and radially expanding the engineered landing zone comprises radially expanding the at least one stent.

In a fifteenth example, in the method of the fourteenth example, the at least one stent is self-expanding, and the step of radially expanding the at least one stent comprises releasing the stent from a constraint to allow the at least one stent to self-expand.

In a sixteenth example, in the method of any on the tenth through fifteenth examples, the frame is self-expanding, and radially expanding the frame comprises releasing the frame from a constraint to allow the frame to self-expand.

In a seventeenth example, in the method of any one of the tenth through sixteenth examples, the engineered landing zone further includes a mobile external coupling, wherein radially expanding the engineered landing zone comprises radially expanding the mobile external coupling, and the method further comprises delivering a branch stent graft to the site such that a portion of the branch stent graft is disposed within the mobile external coupling and a portion of the branch stent graft is disposed within a branch vessel branching from the main vessel, and radially expanding the branch stent graft such that the portion of the branch stent graft disposed within the mobile external coupling engages the mobile external coupling.

In an eighteenth example, in the method of the seventeenth example, the graft material includes an opening disposed therethrough, and longitudinally translating the engineered landing zone frame comprises aligning the mobile external coupling with the opening, and wherein delivering the branch stent graft to the site comprises delivering the branch stent graft through the mobile external coupling and the opening.

In a nineteenth example, in the method of the seventeenth example or the eighteenth example, the branch stent graft is balloon expandable, and radially expanding the branch stent graft comprises expanding a balloon disposed within the branch stent graft.

In a twentieth example, a landing zone prosthesis comprises a frame, an engineered landing zone, and graft material coupled at a first end to the frame and at a second end to the engineered landing zone, wherein the graft material includes a non-stented portion between the frame and the engineered landing zone, wherein in a first configuration the frame and the engineered landing zone are radially compressed with the engineered landing zone longitudinally spaced from the frame, wherein in a second configuration the frame is radially expanded, the engineered landing zone is radially compressed, and the engineered landing zone longitudinally spaced from the frame, and wherein in a third configuration the frame is radially expanded, the engineered landing zone is disposed within the frame and radially expanded, and the frame is secured to a vessel.

In a twenty-first example, the landing zone prosthesis of the twentieth example further comprises endoanchors, wherein in the third configuration, the frame is secured to the vessel via the endoanchors disposed through the frame and into the vessel.

In a twenty-second example, the landing zone prosthesis of the twentieth example or the twenty-first example further includes a fourth configuration wherein the frame is radially expanded, the engineering landing zone is disposed within the frame, and the engineering landing zone is radially compressed.

In a twenty-third example, in the landing zone prosthesis of any one of the twentieth through twenty-second examples, the engineering landing zone comprises at least one stent and landing zone graft material coupled to the at least one stent.

In a twenty-fourth example, in the landing zone prosthesis of the twenty-third example, the landing zone graft material is different than the graft material.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings, which are incorporated herein and form a part of the specification.
FIGS. 1A-1C show various exemplary aneurysms in a patient's aorta.
FIG. 2 shows a side view of a stent graft device according to an embodiment hereof.
FIGS. 3A-3B show side views of embodiments of a main stent portion of the stent graft device of FIG. 2 according to embodiments hereof.
FIG. 4 shows a side view of an embodiment of an engineered landing zone of the stent graft device of FIG. 2 according to an embodiment hereof.
FIGS. 5A-5F show side views of various embodiments of the stent graft device according to embodiments hereof.
FIG. 6 is a block diagram of a method of using the stent graft device of FIG. 2 and FIGS. 5A-5F according to embodiments hereof.
FIG. 7 shows a delivery device, with the stent graft device disposed therein, within an aortic aneurysm of a patient according to embodiments hereof.
FIG. 8 shows the delivery device retracted and the main stent portion of the stent graft device expanded within the aneurysm according to embodiments hereof.
FIG. 9 shows an endoanchor system applying endoanchors to the main stent portion of the stent graft device according to embodiments hereof.
FIG. 10 shows a close-up view of the stent graft device within the aorta of the patient according to embodiments hereof.
FIG. 11 shows the engineered landing zone in a crimped configuration pulled down within the frame of the main stent portion of the stent graft device according to embodiments hereof.
FIGS. 12-13 show the engineered landing zone in an expanded configuration within the frame of the main stent portion of the stent graft device according to embodiments hereof.
FIG. 14 shows a subsequent stent graft device deployed within the engineered landing zone of the stent graft device according to embodiments hereof.
FIG. 15 shows fenestrations within the non-stented portion of the stent graft device according to embodiments hereof.
FIG. 16 shows subsequent branch stent graft devices deployed within the engineered landing zone of the stent graft device of FIG. 15 according to embodiments hereof.
FIG. 17 shows an additional embodiment of the stent graft device according to embodiments hereof.
FIG. 18 shows subsequent branch stent graft devices deployed within the engineered landing zone of the stent graft device of FIG. 17 according to embodiments hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

The terms "proximal" and "distal" herein when used with respect to a delivery system are used with reference to the clinician using the devices. Therefore, "proximal" and "proximally" mean in the direction toward the clinician, and "distal" and "distally" mean in the direction away from the clinician. The terms "proximal" and "distal" herein when used with respect to a stent graft device to be implanted into a body are used with respect to the direction of blood flow. Therefore, "proximal" and "proximally" mean in the upstream or inflow direction, and "distal" and "distally" mean in the downstream or outflow direction.

As used herein, the term "generally" and "substantially" mean approximately. When used to describe angles such as "substantially parallel" or "substantially perpendicular" the term "substantially" means within 10 degrees of the angle. When used to describe shapes such as "substantially" or "generally" cylindrical or "substantially" or "generally" tube-shaped or "generally" or "substantially" conical, the terms mean that the shape would appear cylindrical or tube-shaped or conical to a person of ordinary skill in the art viewing the shape with a naked eye.

Embodiments hereof relate to a stent graft device a main stent portion and an engineered landing zone configured to be deployed at an aortic aneurysm to provide a stable landing zone for stent graft prostheses to be deployed at the aneurysm when the aneurysm lacks a suitable proximal neck for stent graft deployment.

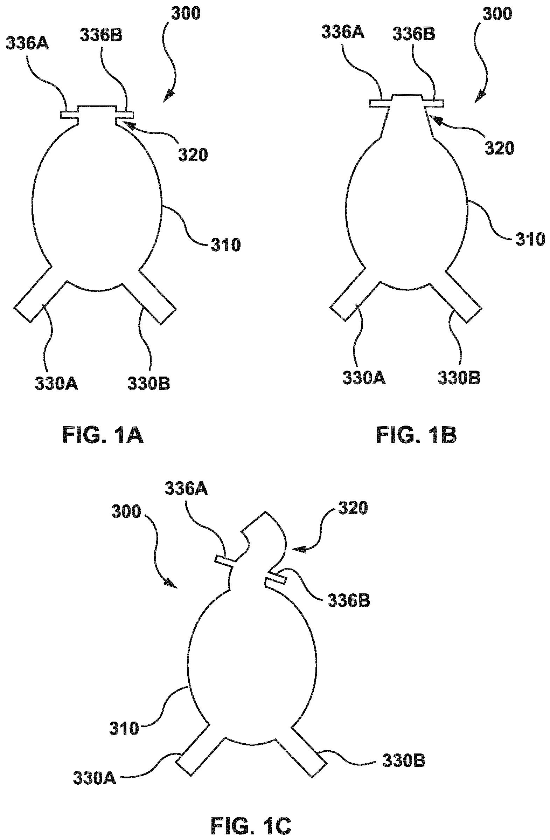

FIGS. 1A-1C depict exemplary aneurysms 300 disposed within an aorta of a patient's vasculature that may be treated using the stent graft device described herein. The aneurysms 300 shown in FIGS. 1A-1C are abdominal aortic aneurysms (AAA) disposed proximal (in FIGS. 1A-1C above) a bifurcation where the aorta splits into first and second iliac arteries 330A, 330B of the patient's vasculature and adjacent renal arteries 336A, 336B branching from the aorta. A wall 310 of the aorta expands due to weakening of the interior wall 310, thereby forming the aneurysm 300. A portion of the aorta directly proximal of the aneurysm 300 is termed a proximal neck 320 of the aneurysm 300. The proximal neck 320 of the aneurysm 300 is known to those skilled in the art as a landing zone for endovascular prostheses that are to be deployed to treat the aneurysms. Depending on the patient's anatomy and size/location of the aneurysm, the proximal neck may not be a suitable landing zone for an endovascular prosthesis. For example, FIG. 1A shows a situation wherein the proximal neck 320 may be too short due to its proximity to the renal arteries 336A, 336B. In another example, FIG. 1B shows a situation wherein the proximal neck 320 angles inwards in a substantially reverse conical shape, rendering the proximal neck 320 unsuitable to accommodate a conventional endovascular stent graft. In other instances, the proximal neck 320 of the aneurysm 300 may be highly angled or curved, making deployment of an endovascular stent graft in those areas difficult, as shown in FIG. 1C. These are examples of hostile neck anatomies, however, other hostile neck anatomies or non-hostile neck anatomies may also be treated using the stent graft device described herein.

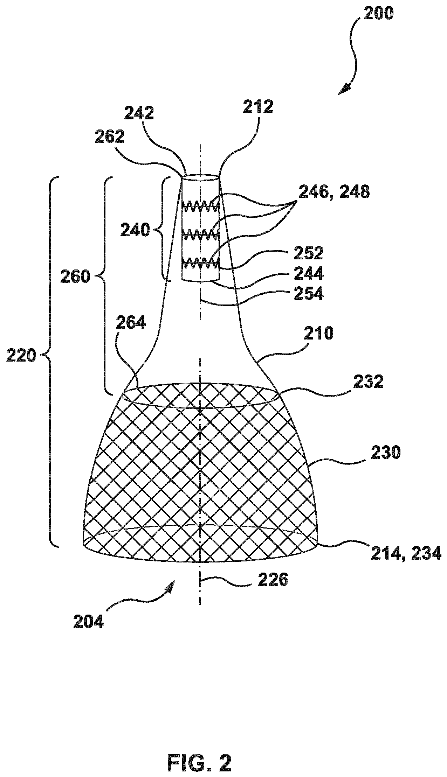

FIGS. 2-5F show a landing zone prosthesis 200 according to embodiments hereof. The landing zone prosthesis 200 is used herein to provide a landing zone for subsequent endovascular prostheses used to bypass an aneurysm 300 of a patient when anatomical constraints, such as the examples mentioned above, are present. The landing zone prosthesis 200 includes a main portion 220 and an engineered landing zone 240. The main portion 220 includes a radially-expandable stent or frame 230 and graft material 210, as explained in more detail below.

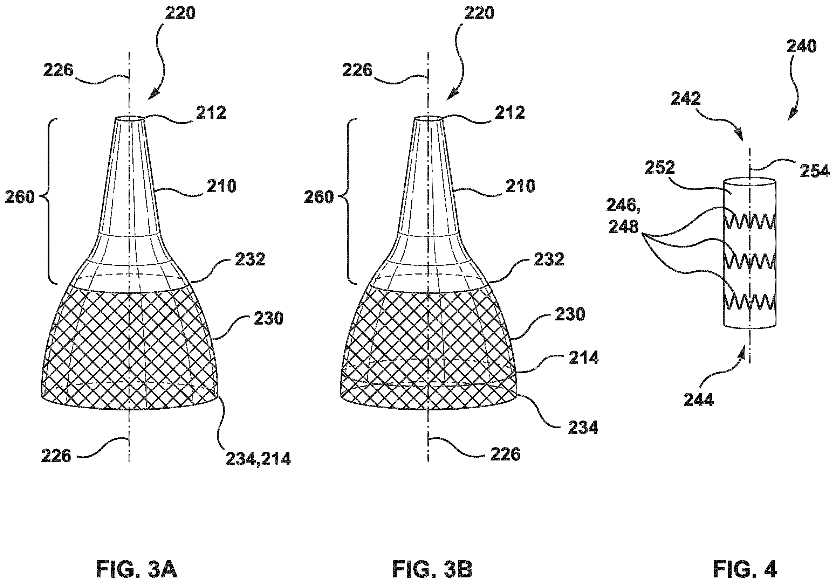

FIGS. 3A-3B show the main portion 220 of the landing zone prosthesis 200. As noted above, the main portion 220 includes a radially-expandable frame 230 and a graft material 210. The frame 230 may be a substantially reverse funnel-shaped stent or scaffold that supports the graft material 210 attached to the frame 230. The frame 230 includes a proximal (upstream) end 232, a distal (downstream) end 234, an interior surface and an exterior surface. The frame 230 as shown includes a plurality of struts that are arranged to form a plurality of side openings or cells arranged circumferentially around a central longitudinal axis of the main portion 220. The frame 230 may be formed in any manner known to those skilled in the art for forming stents. The frame 230 forms a reverse-conical shape when radially expanded and defines central lumen 226 of the main portion 220. The reverse-conical shape conforms to an inner surface of an aneurysm at the proximal neck thereof, as explained in more detail below. The proximal end 232 and the distal end 234 of the frame 230 may have substantially circular cross-sections. The proximal end 232 of the frame 230 has a diameter that is smaller than a diameter of the distal end 234 of the frame 230 such that the diameter of the frame 230 tapers in the proximal direction to create the reverse-funnel shape. The diameter of the proximal end 232 of the frame 230 can range from about 19-65 mm, while the diameter of the distal end 234 of the frame 230 can range from about 19-45 mm. The frame 230 may have a longitudinal length, extending from the proximal end 232 to the distal end 234, of about 20-60 mm. The frame 230 may comprise any appropriate material, such as, but not limited to, stainless steel, nitinol, tantalum, MP35N alloy, platinum, titanium, a chromium-based alloy, a suitable biocompatible alloy, a suitable biocompatible polymer, and/or combinations thereof. The frame 230 may be self-expanding or mechanically-expandable, for example, balloon expandable. The distal end 234 of the frame 230 defines a distal end 204 of the landing zone prosthesis 200.

The graft material 210 may be a thin, elastic, substantially tubular-shaped material that extends from a proximal end 212 to a distal end 214, and includes an interior surface and an exterior surface. The graft material 210 may be coupled to the interior surface or the exterior surface of the frame 230, but in this embodiment it is preferable that the graft material 210 be attached to the interior surface of the frame 230 such that he interior surface of the frame 230 and the exterior surface of the graft material 210 are in direct contact and coupled to one another. The graft material 210 may be coupled to the frame 230 by sutures, adhesives, heat bonding, and/or other coupling mechanisms known to those skilled in the art. The interior surface of the graft material 210 defines a central lumen 226 that extends through the graft material 210 and the frame 230 of the main portion 220 of the landing zone prosthesis 200. The graft material 210 may comprise polyester, ePTFE, silicone, polyurethane, and/or any materials known to those skilled in the art for the purposes described herein, and any combinations of the above.

As shown in FIGS. 3A-3B, a proximal portion of the graft material 210 extends proximally past the proximal end 232 of the frame 230 and is unconstrained by the frame 230. The proximal portion of the graft material 210 that extends proximally past the proximal end 232 of the frame 230 is termed a non-stented or unsupported portion 260 of the main portion 220 of the landing zone prosthesis 200. The non-stented portion 260 does not have any stents or frame directly attached to it.

The frame 230 of the main stent portion 220 can be partially or completely lined with the graft material 210. In embodiments where the frame 230 is completely lined with the graft material 210, the distal end 214 of the graft material 210 aligns with the distal end 234 of the frame 230, as shown in FIG. 3A. In embodiments where the frame 230 is partially lined with the graft material 210, for example, to allow for unconstrained expansion of the main portion 220 to the aortic wall 310, which will be explained in further detail below, a distal portion of the frame 230 can be free of the graft material 210, as shown in FIG. 3B. In other words, the distal end 214 of the graft material 210 terminates proximal to the distal end 234 of the frame 230 such that a distal portion of the frame 230 is not lined with the graft material 210. The distal portion of the frame 230 that is not lined with the graft material 210 can be a region that extends about 0-50 mm from the distal end 234 of the frame 234.

The frame 230 of the main portion 220 includes a radially crimped configuration and a radially expanded configuration. When the frame 230 is in the crimped configuration, the frame 230 is compressed radially to reduce the diameter of the frame 230 such that it can be loaded within a delivery system 100 for transluminal delivery to the site of the aneurysm. The frame 230 of the main portion 220 is then radially expanded to the expanded configuration, shown in FIGS. 3A-3B, such as by self-expansion when the frame 230 is no longer constrained by the delivery system 100. The frame 230 is configured to expand within a proximal portion of the aneurysm 300, which will be described in further detail below.

FIG. 4 shows the engineered landing zone 240 of the landing zone prosthesis 200. The engineered landing zone 240 is a stent graft extending from a proximal end 242 to a distal end 244, and includes a graft material 252 coupled to a plurality of stents 246. The engineered landing zone 240 is configured to provide a stable and secure landing zone or area that an endovascular prosthesis can be deployed within and overlap with, which will be described in greater detail below. The graft material 252 of the engineered landing zone 240 is substantially cylindrical-shaped and defines a central lumen 254 therethrough. The graft material 252 includes a proximal end, a distal end, an interior surface, and an exterior surface. The proximal end of the graft material 252 defines the proximal end 242 of the engineered landing zone 240 and the distal end of the graft material 252 defines the distal end 244 of the engineered landing zone 240, as shown in FIG. 4. The graft material 252 may have a diameter of about 19-32 mm and a longitudinal length, extending from the proximal end to the distal end, of about 10-40 mm, depending on the endovascular prosthesis to be deployed thereafter, which will be discussed in further detail below. The graft material 252 may comprise polyester, ePTFE, silicone, polyurethane, and/or any materials known to those skilled in the art for the purposes described herein, and any combinations of the above.

The plurality of stents 246 of the engineered landing zone 240 are substantially ring shaped and are coupled to the exterior or interior surface of the graft material 252. The stents 246 may be uniformly spaced apart in a longitudinal direction on the exterior surface of the graft material 252, as shown in FIG. 4. Each of the plurality of stents 246 may comprise any appropriate material, such as, but not limited to, stainless steel, nitinol, tantalum, MP35N alloy, platinum, titanium, a chromium-based alloy, a suitable biocompatible alloy, a suitable biocompatible polymer, and/or combinations thereof. The stents 246 of the engineered landing zone 240 may have a zig-zag pattern, as shown in FIG. 4. However, this is not meant to be limiting, as the stents 246 of the engineered landing zone 240 can include any shape or configuration know to those skilled in the art. The stents 246 can be coupled to the exterior or interior surface of the graft material 252 by sutures, adhesives, heat bonding, and/or any other coupling mechanisms known to those skilled in the art.

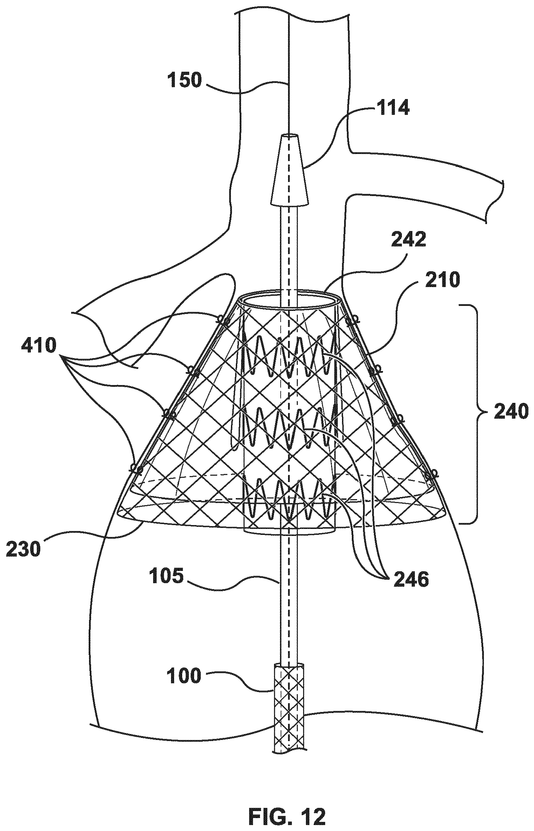

The engineered landing zone 240 includes a radially crimped configuration and a radially expanded configuration. In the crimped configuration, the stents 246 of the engineered landing zone 240 are radially compressed to reduce the diameter of the stents 246. The stents 246 of the engineered landing zone 240 can be held in the radially compressed configuration by diameter reducing ties 248, as shown in FIG. 4. The diameter reducing ties 248 are wires that wrap around the exterior surface of the struts 246 and secure the struts 246 in the crimped configuration prior to deploying the engineered landing zone 240, which will be described in more detail below. When the diameter reducing ties 248 are removed from the struts 246 of the engineered landing zone 240, the engineered landing zone 240 expands to the expanded configuration, as shown in FIG. 12 and described in more detail below. While diameter reducing ties 248 are disclosed for maintaining the engineered landing zone 240 in the radially compressed or crimped configuration, other mechanisms may also be used, such as sheaths (e.g., tear-away).

In the embodiment shown, the engineered landing zone 240 includes exactly three stents 246, however this is not meant to be limiting, as the engineered landing zone 240 can include more or fewer stents 246. For example, the engineered landing zone 240 can include exactly one stent 246, or the engineered landing zone 240 can include exactly two stents 246, or the engineered landing zone 240 can include exactly four stents 246, or the engineered landing zone 240 can include exactly five stents 246, or the engineered landing zone 240 can include more than five stents 246.

The stents 246 of the engineered landing zone 240 are configured such that when they are in the radially expanded configuration, the stent 246 resist further expansion. For example, and not by way of limitation, the stents 246 may be self-expanding such that the radially expanded configuration is a pre-set configuration of the stents 246. Thus, when released from the diameter reducing ties, the stents 246 will radially expand to the pre-set (radially expanded) configuration. Further, if a force, such as from an endovascular prosthesis disposed within the engineered landing zone 240, attempts to further radially expand the stents 246, the stents 246 will resist such a force to attempt to remain in the pre-set configuration. In another embodiment, the graft material 252 is not expandable in that it folds in the radially compressed configuration of the engineered landing zone 240 are unfolds in the radially expanded configuration of the engineered landing zone 240. The diameter of the graft material 252 in the radially expanded is set such as to resist further expansion by an endovascular prosthesis disposed therein, as explained in more detail below.

The proximal end 212 of the graft material 210 also defines the proximal end of the non-stented portion 260 of the main portion 220. Further, where the graft material 210 attaches to the proximal end 232 of the frame 230 defines a distal end 264 of the non-stented portion 260. The proximal end 212 of the graft material 210 is attached to the engineered landing zone 240, as described in more detail below. The non-stented portion 260 of the stent graft device 200 is configured to sock, or invert, to allow the engineered landing zone 240 to be pulled into position within the lumen 226 of the frame 230 of the main portion 220, which will be explained in more detail below. The non-stented portion 260 can have a longitudinal length, extending from the proximal end 212 of the graft material 210 to the distal end 264 of the non-stented portion 260, of at least 5 mm, or at least 10 mm, or at least 20 mm, or at least 40 mm, or at least 60 mm, or at least 80 mm, depending on the amount of inversion desired, the amount of graft material 210 desired between the engineered landing zone 240 and the frame 230 when inverted, and/or the location of the connection between the graft material 210 and the engineered landing zone 240. The longitudinal length of the non-stented portion 260 may also be at least 50%, at least 75%, at least 100%, at least 150%, or at least 200% the length of the engineered landing zone 240. The longitudinal length of the non-stented portion 260 may also be at least 2 times, at least 3 times, at least 5 times, or at least 10 times a longitudinal length between adjacent stents 246 of the engineered landing zone 240.

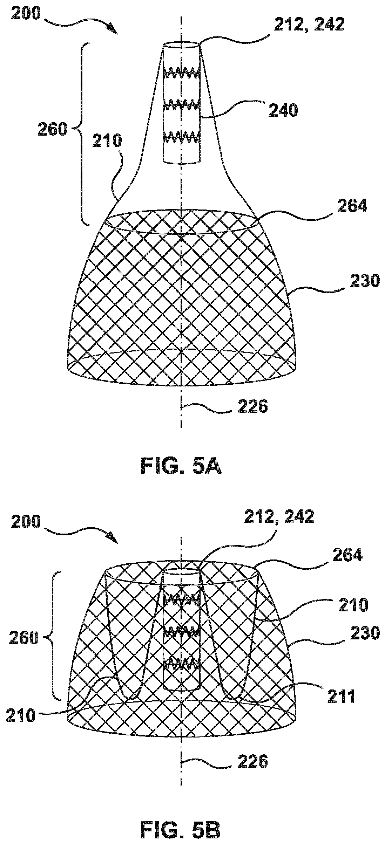

FIGS. 5A-5F show various configurations of the landing zone prosthesis 200 according to embodiments hereof. For example, FIGS. 5A-5B show landing zone prosthesis 200 with the proximal end 212 of the graft material 210 coupled to the proximal end 242 of the engineered landing zone 240. When the engineered landing zone 240 is pulled into the lumen 226 of the frame 230, the graft material 210 of the non-stented portion 240 socks or inverts at the connection to the frame 230. Also in such a configuration, when the engineered landing zone 240 is pulled into the frame 230, a second fold 211 in the non-stented portion 260 of the graft material 210 is formed, thereby resulting in a double layer of graft material 210 surrounding the exterior surface of the engineered landing zone 240, as seen in FIG. 5B.

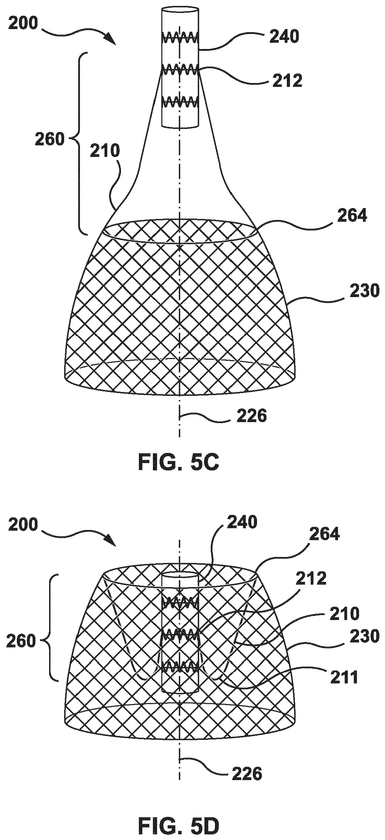

FIGS. 5C-5D show an embodiment of the landing zone prosthesis 200 wherein the proximal end 212 of the graft material 210 is coupled to the exterior surface of the engineered landing zone 240 between the proximal and distal ends 242, 244 of the engineered landing zone 240. In such a configuration, a distal portion of the engineered landing zone 240 is disposed within the central lumen 226 of the non-stented portion 260 of the landing zone prosthesis 200 and a proximal portion of the engineered landing zone 240 is free of the graft material 210 and is disposed proximal to the proximal end 212 of the graft material 210. When the engineered landing zone 240 is pulled into the lumen 226 of the frame 230, as shown in FIG. 5D, the graft material 210 of the non-stented portion 240 to socks or inverts at the connection to the frame 230. Also in such a configuration, when the engineered landing zone 240 is pulled into the frame 230, a second fold 211 in the non-stented portion 260 of the graft material 210 is formed, thereby resulting in a double layer of graft material 210 surrounding the exterior surface of the engineered landing zone 240 for a portion engineered landing zone 240, and a portion that is not double layered, as shown in FIG. 5D.

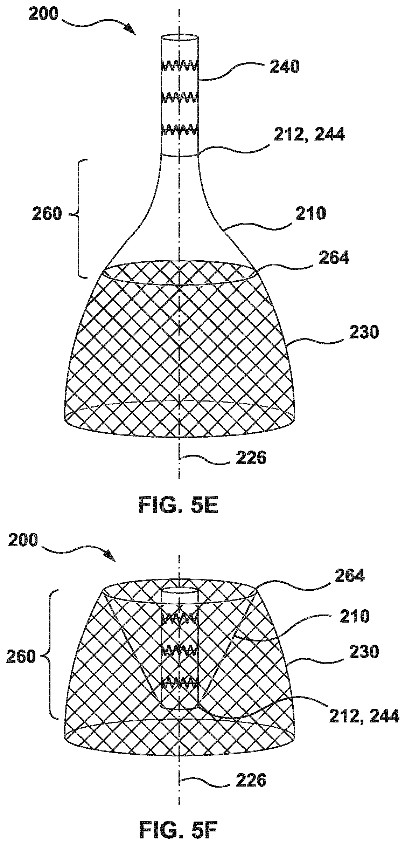

FIGS. 5E-5F show an embodiment of the landing zone prosthesis 200 wherein the proximal end 212 of the graft material 210 is coupled to the distal end 244 of the engineered landing zone 240. In such a configuration, the entire engineered landing zone 240 is disposed proximal to the non-stented portion 260 of the main portion 220 of the landing zone prosthesis 200. When the engineered landing zone 240 is pulled into the lumen 226 of the frame 226, as shown in FIG. 5F, the graft material 210 of the non-stented portion 240 socks or inverts. Because the proximal end 212 of the graft material 210 is coupled to the distal end 244 of the engineered landing zone 240, when the engineering landing zone is inverted, it results in single layer of the graft material 210 of the non-stented portion 240 between the engineering landing zone 240 and the graft material 210 attached to the frame 230.

In the embodiment described above, although the graft material 210 and the graft material 252 of the engineered landing zone are described as different graft material, this is not meant to be limiting. In embodiments, the graft material 252 of the engineered landing zone 240 may be a continuation of the graft material 210, especially in the situations where the graft material 210 is coupled to the proximal end or the distal end of the engineered landing zone 240.

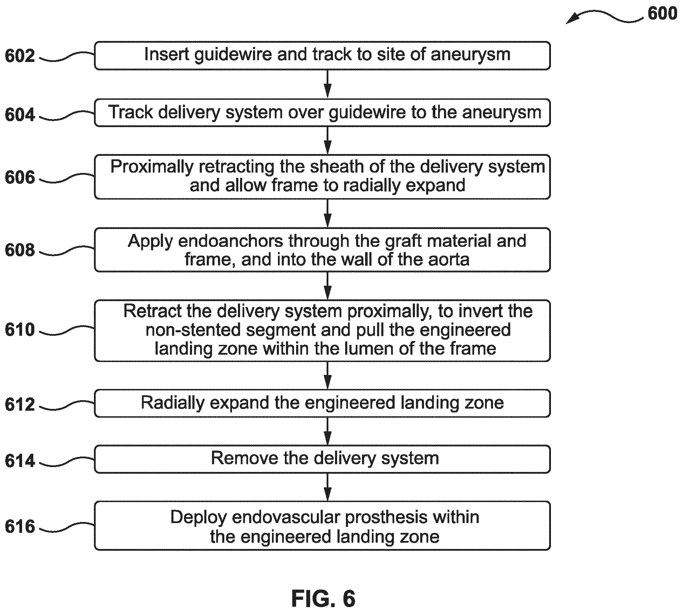

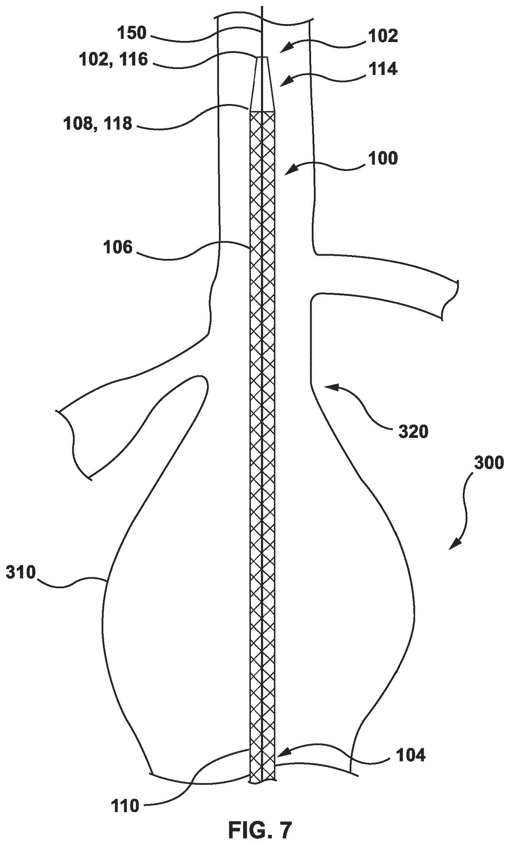

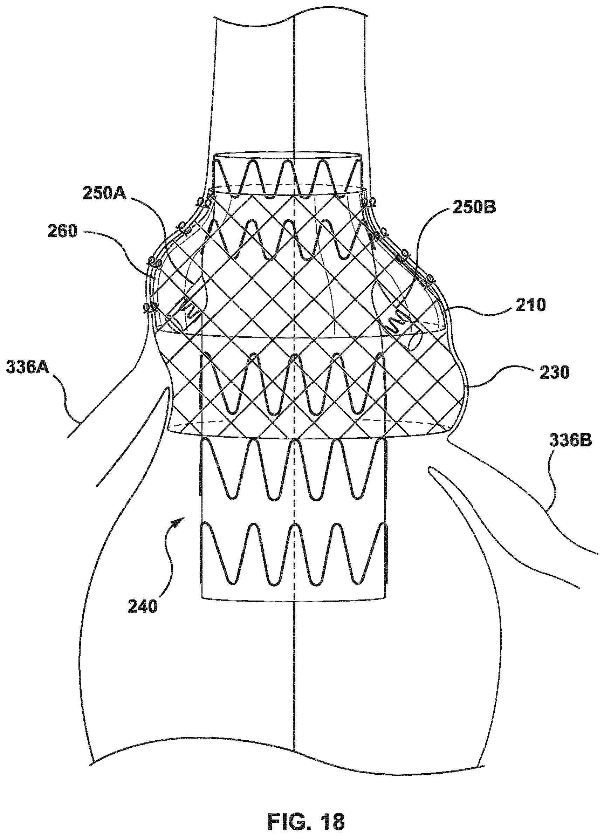

FIG. 6 shows block diagram of a method 600 of delivering and deploying the landing zone prosthesis 200 and a subsequent endovascular prosthesis, and FIGS. 7-18 illustrate steps of the method, with variations. It should be understood that the method is not limited to the landing zone prosthesis 200 shown, and other landing zone prostheses and variations thereof as described herein can be used in the method. Further, additional or fewer steps of the method described may be used, and some steps may be omitted in this description as being known to those skilled in the art. The method described herein is described with respect to delivering the landing zone prosthesis 200 within an abdominal aortic aneurysm (AAA) of a patient, however, this is not meant to be limiting, and the landing zone prosthesis 200 can be used to treat thoracic aortic aneurysms (TAA) as well.

In a preliminary step, the landing zone prosthesis 200 is loaded within a delivery system 100 in a radially compressed configuration. In an embodiment, the landing zone prosthesis is loaded within a sheath 106 of the delivery system 100 in the radially compressed configuration. Although a particular delivery system 100 is shown and partially described, it is not meant to be limiting, and other delivery systems suitable for use with endovascular prostheses may be utilized. A brief description of some of the parts of the delivery system 100 will be provided herein for context, but are not mean to be limiting. For example, the delivery system 100 may include a distal end 102, a proximal end 104, an inner shaft 105, a sheath 106, and a nosecone 114. The sheath 106 is a tubular structure that includes a distal end 108, a proximal end 110, an exterior surface and an interior surface that defines a lumen 112 configured to house the landing zone prosthesis 200 in the crimped configuration. The proximal end 110 of the sheath 106 is coupled to a handle (not shown) that allows a user to hold and control the delivery system 100 as needed. The handle (not shown) may include an actuator (not shown) to retract the sheath 106 to uncover the landing zone prosthesis 200, thereby enabling self-expansion of the landing zone prosthesis. The nosecone 1114 may be coupled to the inner shaft 105. Further, the inner shaft 105 may include a guidewire lumen 107. When loaded into the delivery system 100, the landing zone prosthesis 200 may be coupled to the delivery system 100 via a retainer 130, as known to those skilled in the art. For example, and not by way of limitation, the retainer 130 may be coupled to the inner shaft, the nosecone, or a middle shaft (not shown) extending between the inner shaft and the sheath 106. The nosecone 114 is a distally tapered element that includes a distal end 116, a proximal end 118, an exterior surface and an interior surface that defines a lumen 120 extending from the distal end 116 to the proximal end 118 that allows passage of a guidewire therethrough.

When the landing zone prosthesis 200 is loaded within the central lumen 112 of the cover 106 of the delivery system 100, both the frame 230 of the main portion 220 and the stents 246 of the engineered landing zone 240 are in the radially compressed configuration. The landing zone prosthesis 200 is loaded into the lumen 112 of the sheath 106 and over the inner shaft 105 and the middle shaft (not shown), if the delivery system 100 includes them. The proximal end 102 of the landing zone prosthesis 200 is disposed adjacent the proximal end 118 of the nosecone 114, and may be coupled to the retainer 130. It is noted that the proximal end 102 of the landing zone prosthesis 200 is located at the distal end of the delivery system 100 because, as explained above, the terms proximal and distal are used differently with respect to the landing zone prosthesis 200 and the delivery system 100.

Returning to FIG. 6 and the method 600, in a first step 602 of the method 600, a guidewire 150 is inserted within one of the iliac arteries 330A, 330B and is tracked (in an upstream direction (upwards FIG. 7) towards the abdominal aortic aneurysm 300 within the aorta of the patient, as shown in FIG. 7. In an embodiment, as known to those skilled in the art, a distal end of the guidewire 150 introduced through one of the femoral arteries, and is tracked within the one of the iliac arteries 330A, 330B and into the aorta of the patient. The distal end of the guidewire 150 is tracked through the aneurysm 300 to a location upstream of the aneurysm 300.

In a step 604 of the method 600, the delivery system 100 with the landing zone prosthesis 200 disposed therein is tracked over the guidewire 150 to the location of the aneurysm 300. In an embodiment, the delivery system 100 is tracked such that the proximal end 232 of the frame 230 is aligned with the proximal neck 320 of the aneurysm 300. When tracked to such a location the engineered landing zone 240 and the nosecone are located upstream of the aneurysm 300, as shown in FIG. 7. The delivery system 100 may be tracked over the guidewire 150 as known to those skilled in the art. For example, and not by way of limitation, a proximal end of the guidewire 150 may be inserted into the lumen 120 of the nosecone 114 and through the guidewire lumen 107 of the inner shaft 105. The delivery system 100 may be an over-the-wire system or a rapid exchange system. The delivery system 100 is then tracked over the guidewire to the aneurysm 300, as described above.

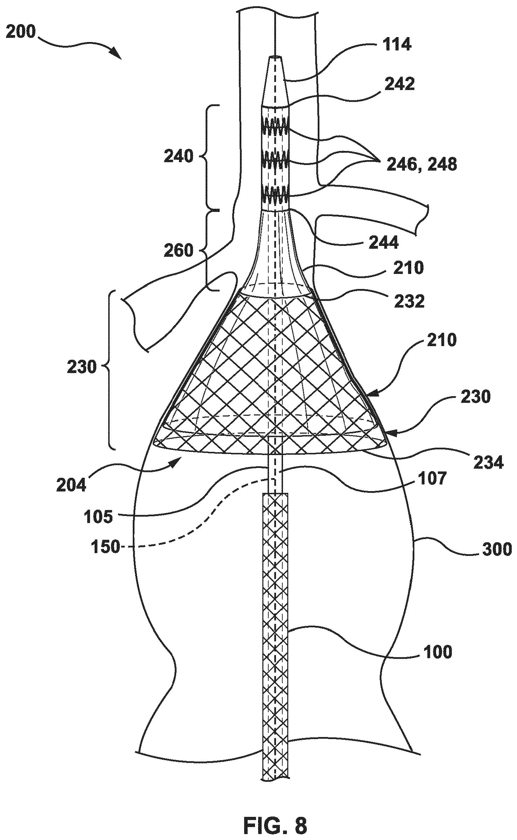

In a next step 606 of the method 600, the sheath 106 of the delivery system 100 is retracted proximally to expose the landing zone prosthesis 200 within the aorta of the patient. For example, an actuator located at the handle of the delivery system 100 may be actuated to retract the sheath 106. When the sheath 106 is retracted, the frame 230 radially expands within the proximal neck region 320 of the aneurysm 300, as shown in FIG. 8. The reverse funnel shape of the frame 230 allows the frame 230 to contact the aneurysm 300 when there is little to no proximal neck 320. Further, the engineered landing zone 240 remains in the radially compressed configuration through the use of diameter reducing ties as discussed above and/or the retainer 130 and/or devices to keep the engineered landing zone 240 radially compressed (e.g., a split sheath could be used with a portion extending proximally from the nosecone 114 and covering the engineered landing zone 240). The graft material 210 of non-stented portion 260 extends from the radially expanded proximal end 232 of the frame 230 to the radially compressed engineered landing zone 240. In the radially expanded configuration, the frame 230 expands to contact the wall 310 of the aneurysm 300.

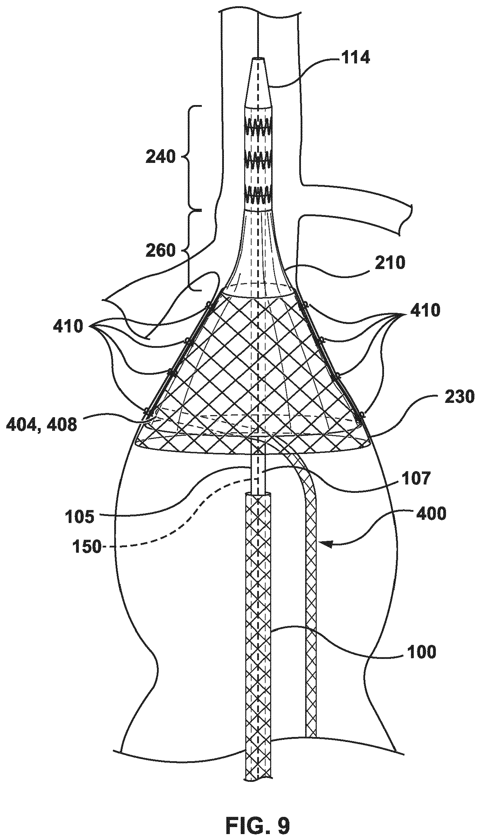

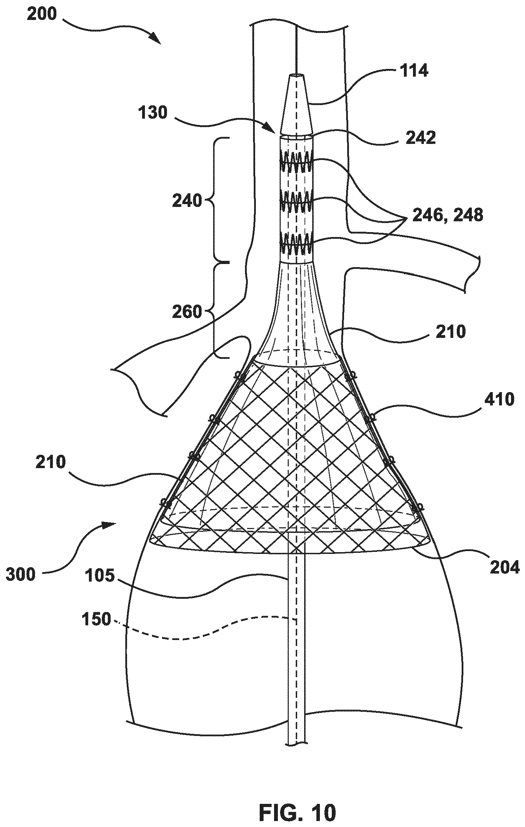

Although the frame 230 radially expands against the wall 310 of the aneurysm 300, it is not desirable to apply excess outward pressure to the wall 310 of the aneurysm 300. Therefore, the frame 230 is preferably not retained in the aorta through outward pressure as many conventional stent graft prostheses are. Instead, in a next step 608 of the method 600, a plurality of endoanchors 410 are applied at various locations through the frame 230 and into the wall 310 to secure the frame 230 and the graft material 210 that lines the frame 230 to the wall 310, as shown in FIG. 9. In embodiments of the step 608, an endoanchor system 400 may be introduced through one of the femoral arteries and tracked retrograde to the location of the aneurysm or may be introduced via supra-aortic access and tracked antegrade to the location of the aneurysm. The endoanchor system 400 may include a steerable sheath 402 and a handle with an actuator (not shown). The endoanchor system 400 further includes a tip 408 disposed at a distal end 404 of the steerable sheath 402 configured to implant the endoanchors 410 through the graft material 210 and frame 230 of the main portion 220 and into the wall 310 of the aneurysm 300. The endoanchors 410 may be substantially helicalshaped segments that may be screwed through the graft material 210, through cells of the frame 230, and into the wall 310 of the aneurysm 300 by the actuator on the handle. The endoanchors 410 physically attach the landing zone prosthesis 200 to the vasculature, and therefore stabilizes the engineered landing zone 240, as described in more detail below. Any suitable number of endoanchors 410 may be applied to effectively secure the main portion 220 of the landing zone prosthesis 200 to the aneurysm 300, as shown in FIG. 10. An example of an endoanchor system 400 that may be used is the Heli-Fx^{™} EndoAnchor^{™} System available from Medtronic, Inc. Other similar systems may also be used. Alternatively, adhesive may be used to secure the graft material 210 and/or the frame 230 to the wall of the aneurysm 300.

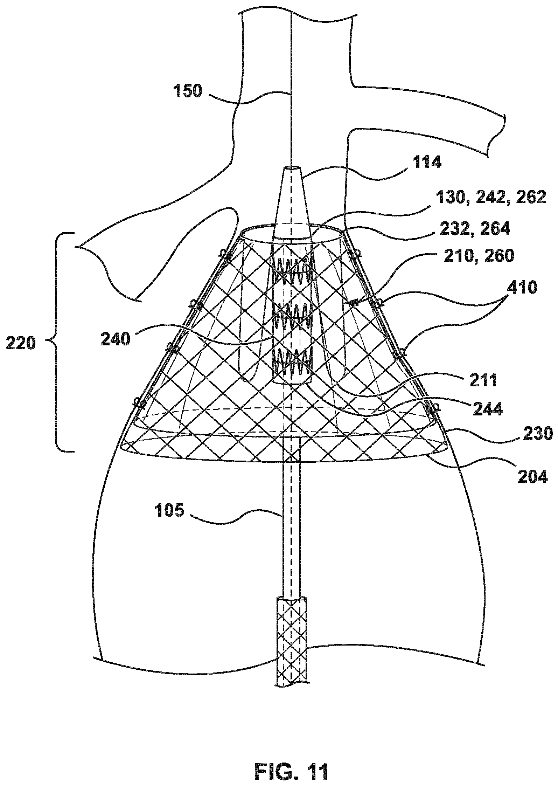

In another step 610 of the method 600, the delivery system 100 is retracted proximally. Such retraction can be accomplished via pulling the handle of the delivery system 100. As the delivery system 100 is retracted, the inner shaft 105 and the nosecone 114 coupled thereto also retract. Such retraction of the inner shaft 105 and nosecone 114, also pulls the engineered landing zone 240 proximally (downstream in this instance) due to the engineered landing zone 240 being coupled to the retainer 130, being disposed within a distal sheath, and/or the nosecone 114 exerting pressure against the engineered landing zone 240. As the engineered landing zone 240 is retracted proximally by the delivery system 100, the proximal end 212 of the graft material 210, which is part of the non-stented portion 260 and is coupled to the engineered landing zone 240, also translates proximally (downstream) such that the non-stented portion 260 begins to fold inward in an inverting or socking motion, as shown in FIG. 11. In other words, as the delivery system 100 is retracted proximally, the engineered landing zone 240 is pulled into the frame 230 and the graft material 210 of the non-stented portion 260 folds inwardly around the exterior surface of the engineered landing zone 240. The distal end 264 of the non-stent portion 260 (i.e., where the graft material 210 is attached to the proximal end 232 of the frame 230) does not translate as it is attached to the frame 230. Instead, as the engineered landing zone 240 is pulled proximally past the distal end 264 of the non-stented portion 260 of the graft material 210, the graft material 210 rotates about the connection to the frame 230, thereby inverting such that the non-stent portion 260 extends downstream from the proximal end 232 of the frame 230. Further, the engineered landing zone 240 is preferably pulled to a location such that the proximal end 242 of the engineered landing zone 240 is aligned with the proximal end 232 of the frame 230, though this is not required.

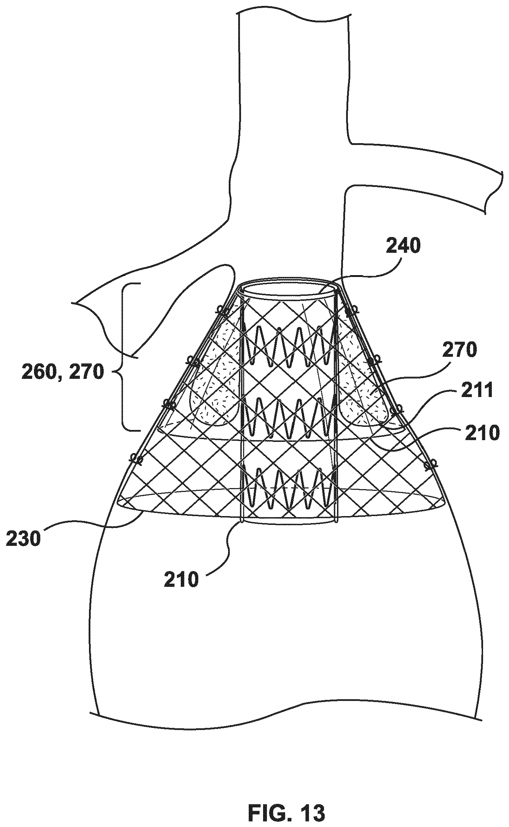

As noted above with respect to FIGS. 5A-5F, depending on the location where the graft material 210 of the non-stented portion 260 is coupled to the engineered landing zone 240, the graft material 210 may include more than one fold. For example, the graft material 210 folds or inverts at the proximal end 232 of the frame 230. As shown, for example in FIGS. 5B, 5C, 11 and 13, the graft material 210 may include an additional fold 211 such that there are two layers of the non-stent portion 260 of the graft material 210 between the engineered landing zone 240 and the frame 230. In such an embodiment, the graft material may create a pocket 270 that may fill with thrombosed blood or a sac filling material to help support sealing at the neck of the aneurysm 300, as shown in FIG. 13.

With the engineered landing zone 240 located within the frame 230, a step 612 of the method 600 comprises radially expanding the engineered landing zone 240. In an embodiment, the engineered landing zone 240 may be radially expanded by releasing diameter reducing ties, such as by removing a trigger wire to release the diameter reducing ties, as known to those skilled in the art. With the diameter reducing ties released, the stents 246 of the engineered landing zone 240 self-expand to radially expand the engineered landing zone 240. Self-expansion of the engineered landing zone 240 may release the engineered landing zone 240 from the retainer 130 or the retainer 130 may be moved to release the engineered landing zone 240. In other embodiments, a distal sheath coupled to the nosecone 114 may be moved upstream to uncover engineering landing zone 240. Other embodiments for radially expanding the engineering landing zone 240 may also be used, such as, but not limited to, balloon expansion.

In a next step 614 of the method 600, the delivery system 100 can be removed from the vasculature by pulling the handle, which will pull the sheath 106, the inner shaft 105 and the nosecone 114 coupled thereto, and other parts of the delivery system 100. The guidewire 150 may remain in place.

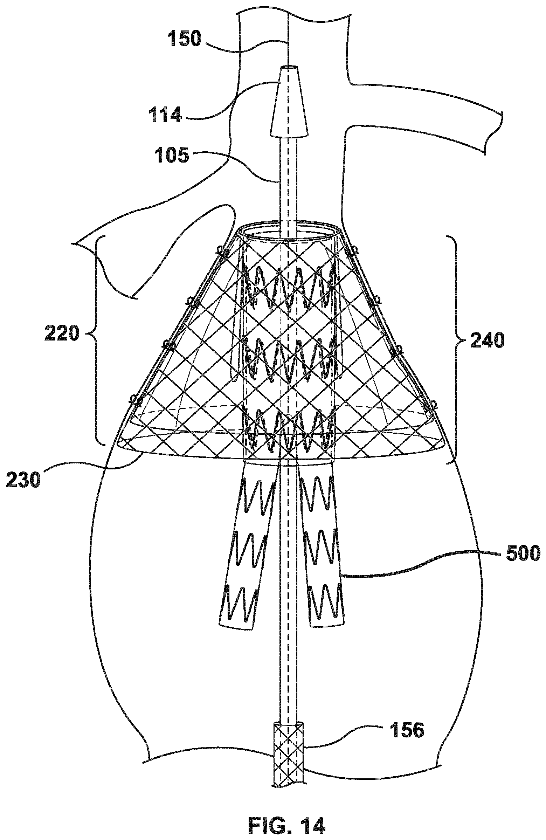

With the delivery system 100 removed, the landing zone prosthesis 200 is in the desirable position to receive a subsequent endovascular prosthesis such as a stent graft prosthesis for bypassing the aneurysm 300. In a next step 616 of the method 600, such an endovascular prosthesis 600 may be delivered and deployed within the engineered landing zone 240 of the landing zone prosthesis 200, as shown in FIG. 14. The endovascular prosthesis 500 may be delivered by a second delivery system 150, as shown in FIG. 14. The additional endovascular prosthesis 500 is delivered such that a proximal portion thereof is disposed within the engineered landing zone 240. The additional endovascular prosthesis 500 is then radially expanded within the engineered landing zone 240, such as by retracting a sheath 156 of the second delivery system 150 or other radial expansion methods (e.g. balloon expansion). The engineered landing zone 240 provides support for the additional endovascular prosthesis 500 to expand against to maintain its position in the vasculature, replacing a landing zone of the aorta in situations where a landing zone is insufficient, as described above. The additional endovascular prosthesis 500 can be any endovascular prosthesis suitable for use to bypass an aneurysm, as known to those skilled in the art.

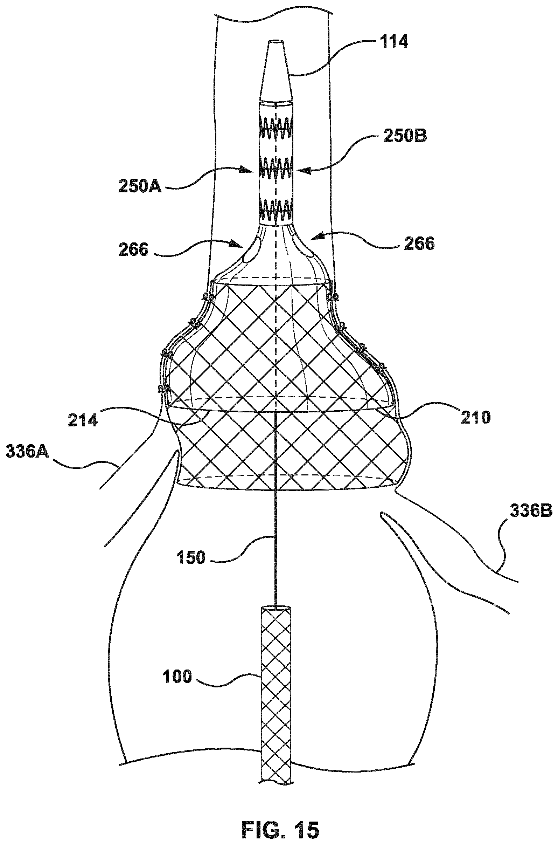

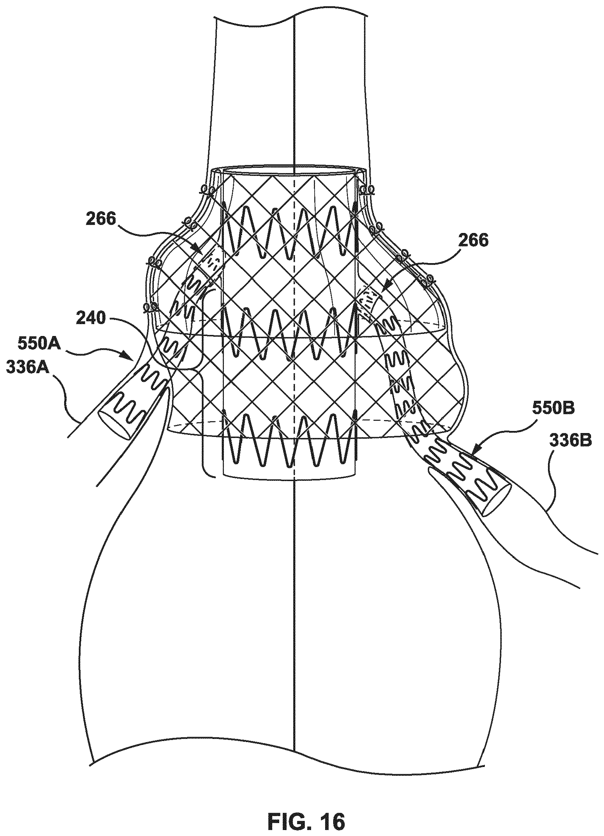

Although described above with respect to a short neck and/or conical neck aneurysm, in some circumstances, the landing zone prosthesis 200 may have to cross branch vessels of the aorta, for example, the renal arteries 336A, 336B. In such a situation, the frame 230 may be deployed further upstream in the aorta, as shown in FIG. 15. Further, the distal end 214 of the graft material 210 terminates proximal of the branch vessels 336A, 336B, as also shown in FIG. 15, so as to provide access to the branch vessels 336A, 336B. Further, the non-stented portion 360 of the graft material includes openings 266, as also shown in FIG. 15. When the engineering landing zone 240 is retracted into the frame 230, there still needs to be access through the engineered landing zone 240 to the openings 266. In an embodiment, as shown in FIG. 15, the engineering landing zone 240 includes couplings, such as mobile external couplings 250A, 250B, that are deployed adjacent to or through the openings 266 when the engineered landing zone 240 is radially expanded. In embodiments, the mobile external coupling may be as described, for example, in U.S. Patent Nos. 8,333,800; 8,337,546; 8,506,622; 8,540,764; and 9,839,542 . With the engineering landing zone 240, including the mobile external couplings 250A, 250B, radially expanded, side branch stent grafts 550A, 550B may be deployed through the mobile external couplings 250A, 250B, through the openings 266, and into the branch vessels 336A, 336B, as shown in FIG. 16. The openings 266 and mobile external couplings 250A, 250B need not be perfectly aligned with the side branch vessels 336A, 336B, as shown in FIG. 16.

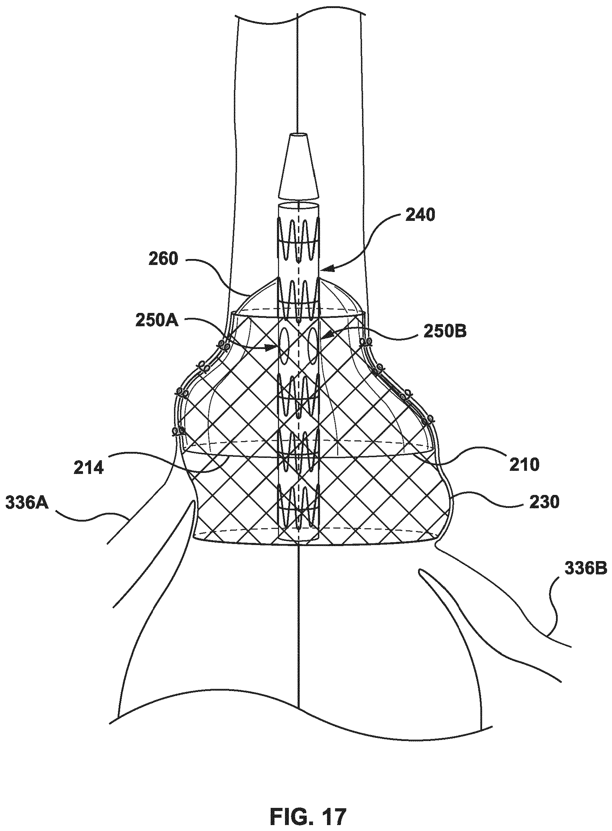

In another embodiment, similar to the embodiment of FIGS. 15-16, the landing zone device 200 includes a shortened non-stented portion 260 wherein the distal end 262 of the non-stented portion 260 coupled to a distal region of the engineered landing zone 240 so as to reduce the distance the engineered landing zone 240 can translate within the frame 230 of the main portion 220. The shortened non-stented portion 260 is such that openings 266 in the non-stented portion 260 of the graft material 210 are not needed, as the non-stented portion 260 of the graft material 210 will not block access from within the engineered landing zone 240 to the branch vessels 336A, 336B. Thus, as shown in FIGS. 17-18, the mobile external couplings 250A, 250B of the engineered landing zone 240 provide access to the branch vessels 336A, 336B. Side branch stents graft such as the side branch stent grafts 550A, 550B shown in FIG.16, may then be deployed through the mobile external couplings 250A, 250B and into the side branch vessels 336A, 336B.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques).

## Claims

1. A landing zone prosthesis comprising:
a frame (230);
an engineered landing zone (240); and
graft material (210) coupled at a first end to the frame and at a second end to the engineered landing zone, wherein the graft material includes a non-stented portion (260) between the frame and the engineered landing zone;
wherein in a first configuration the frame and the engineered landing zone are radially compressed with the engineered landing zone longitudinally spaced from the frame,
wherein in a second configuration the frame is radially expanded, the engineered landing zone is radially compressed, and the engineered landing zone longitudinally spaced from the frame, and
wherein in a third configuration the frame is radially expanded, the engineered landing zone is disposed within the frame and radially expanded, and the frame is configured to be secured to a vessel.

2. The landing zone prosthesis of claim 1, further comprising endoanchors (410), wherein in the third configuration, the frame is configured to be secured to the vessel via the endoanchors disposed through the frame and into the vessel.

3. The landing zone prosthesis of claim 2, wherein the graft material lines at least a portion of the frame, and wherein in the third configuration, the endoanchors are configured to be deployed through the graft material.

4. The landing zone prosthesis of any preceding claim, wherein in a fourth configuration the frame is radially expanded, the engineering landing zone is disposed within the frame, and the engineering landing zone is radially compressed.

5. The landing zone prosthesis of any preceding claim, wherein the engineering landing zone comprises at least one stent (246) and a landing zone graft material (252) coupled to the at least one stent.

6. The landing zone prosthesis of claim 5, wherein in the third configuration the engineered landing zone is radially expanded by radially expanding the at least one stent.

7. The landing zone prosthesis of claim 5 or 6, wherein the at least one stent is self-expanding, and wherein the at least one stent is configured to self-expand by releasing the stent from a constraint.

8. The landing zone prosthesis of any preceding claim, wherein the landing zone graft material is different than the graft material.

9. The landing zone prosthesis of any one of claims 1 to 7, wherein the landing zone graft material is an extension of the graft material.

10. The landing zone prosthesis of any preceding claim, wherein the frame is self-expanding, and wherein the frame is configured to self-expand by releasing the frame from a constraint.

11. The landing zone prosthesis of any preceding claim, wherein in the third configuration, the frame is secured to the vessel via adhesively securing the frame and/or the graft material attached to the frame to the vessel.

12. The landing zone prosthesis of any preceding claim, wherein the engineered landing zone further includes a mobile external coupling (250A, 250B) that is configured to be deployed when the engineered landing zone (240) is radially expanded.

13. A system comprising the landing zone prosthesis of claim 12 and a branch stent graft (550A, 550B) configured such that a portion of the branch stent graft can be disposed within a branch vessel branching from the vessel, and wherein a portion of the branch stent graft is disposed within the mobile external coupling and engages the mobile external coupling.

14. The system of claim 13, wherein the branch stent graft is balloon expandable, and the branch stent graft is configured to radially expand by expanding a balloon disposed within the branch stent graft.

15. The system of claim 13 or 14, wherein the graft material includes an opening (266) disposed therethrough, and the mobile external coupling is configured to be deployed adjacent to or through the opening (266); and optionally wherein the branch stent graft is configured to be delivered to the branch vessel by delivering the branch stent graft through the mobile external coupling and the opening.

## Patentansprüche

1. Landungzonenprothese, umfassend:
einen Rahmen (230);
eine konstruierte Landungzone (240); und
Transplantatmaterial (210), das an einem ersten Ende mit dem Rahmen und an einem zweiten Ende mit der konstruierten Landungzone gekoppelt ist, wobei das Transplantatmaterial einen nicht gestenteten Abschnitt (260) zwischen dem Rahmen und der konstruierten Landungzone einschließt;
wobei in einer ersten Konfiguration der Rahmen und die konstruierte Landungzone radial komprimiert sind, wobei die konstruierte Landungzone in Längsrichtung von dem Rahmen beabstandet ist,
wobei in einer zweiten Konfiguration der Rahmen radial expandiert ist, die konstruierte Landungzone radial komprimiert ist und die konstruierte Landungzone in Längsrichtung von dem Rahmen beabstandet ist, und
wobei in einer dritten Konfiguration der Rahmen radial expandiert ist, die konstruierte Landungzone innerhalb des Rahmens angeordnet und radial expandiert ist und der Rahmen konfiguriert ist, um an einem Gefäß gesichert zu werden.

2. Landungzonenprothese nach Anspruch 1, ferner umfassend Endoanker (410),
wobei in der dritten Konfiguration der Rahmen konfiguriert ist, um über die Endoanker, die durch den Rahmen und in das Gefäß angeordnet sind, an dem Gefäß gesichert zu werden.

3. Landungzonenprothese nach Anspruch 2, wobei das Transplantatmaterial mindestens einen Abschnitt des Rahmens auskleidet, und wobei in der dritten Konfiguration die Endoanker konfiguriert sind, um durch das Transplantatmaterial hindurch entfaltet zu werden.

4. Landungzonenprothese nach einem der vorstehenden Ansprüche, wobei in einer vierten Konfiguration der Rahmen radial expandiert ist, die konstruierte Landungzone innerhalb des Rahmens angeordnet ist und die konstruierte Landungzone radial komprimiert ist.

5. Landungzonenprothese nach einem der vorstehenden Ansprüche, wobei die konstruierte Landungzone mindestens einen Stent (246) und ein Landungzonentransplantatmaterial (252), das mit dem mindestens einen Stent gekoppelt ist, umfasst.

6. Landungzonenprothese nach Anspruch 5, wobei in der dritten Konfiguration die konstruierte Landungzone durch radiales Expandieren des mindestens einen Stents radial expandiert wird.

7. Landungzonenprothese nach Anspruch 5 oder 6, wobei der mindestens eine Stent selbstexpandierend ist, und wobei der mindestens eine Stent konfiguriert ist, um durch Lösen des Stents aus einer Einspannung selbst zu expandieren.

8. Landungzonenprothese nach einem der vorstehenden Ansprüche, wobei sich das Landungzonentransplantatmaterial von dem Transplantatmaterial unterscheidet.

9. Landungzonenprothese nach einem der Ansprüche 1 bis 7, wobei das Landungzonentransplantatmaterial eine Erstreckung des Transplantatmaterials ist.

10. Landungzonenprothese nach einem der vorstehenden Ansprüche, wobei der Rahmen selbstexpandierend ist, und wobei der Rahmen konfiguriert ist, um durch Lösen des Rahmens aus einer Einspannung selbst zu expandieren.

11. Landungzonenprothese nach einem der vorstehenden Ansprüche, wobei in der dritten Konfiguration der Rahmen über klebend Sichern des Rahmens und/oder des Transplantatmaterials, das an dem Rahmen angebracht ist, an dem Gefäß gesichert ist.

12. Landungzonenprothese nach einem der vorstehenden Ansprüche, wobei die konstruierte Landungzone ferner eine mobile äußere Kopplung (250A, 250B), die konfiguriert ist, um entfaltet zu werden, wenn die konstruierte Landungzone (240) radial expandiert wird, einschließt.

13. System, umfassend die Landungzonenprothese nach Anspruch 12 und ein Zweigstenttransplantat (550A, 550B), das derart konfiguriert ist, dass ein Abschnitt des Zweigstenttransplantats innerhalb eines Zweiggefäßes, das von dem Gefäß abzweigt, angeordnet werden kann, und wobei ein Abschnitt des Zweigstenttransplantats innerhalb der mobilen äußeren Kopplung angeordnet ist und die mobile äußere Kopplung in Eingriff nimmt.

14. System nach Anspruch 13, wobei das Zweigstenttransplantat ballonexpandierbar ist und das Zweigstenttransplantat konfiguriert ist, um durch Expandieren eines Ballons, der innerhalb des Zweigstenttransplantats angeordnet ist, radial zu expandieren.

15. System nach Anspruch 13 oder 14, wobei das Transplantatmaterial eine dahindurch angeordnete Öffnung (266) einschließt und die mobile externe Kopplung konfiguriert ist, um angrenzend an oder durch die Öffnung (266) entfaltet zu werden; und optional wobei das Zweigstenttransplantat konfiguriert ist, um an das Zweiggefäß durch Abgeben des Zweigstenttransplantats durch die mobile äußere Kopplung und die Öffnung abgegeben zu werden.

## Revendications

1. Prothèse de zone d'implantation comprenant :
une armature (230) ;
une zone d'implantation artificielle (240) ; et
un matériau de greffon (210) accouplé au niveau d'une première extrémité à l'armature et au niveau d'une seconde extrémité à la zone d'implantation artificielle, dans laquelle le matériau de greffon comporte une partie sans endoprothèse (260) entre l'armature et la zone d'implantation artificielle ;
dans laquelle dans une première configuration l'armature et la zone d'implantation artificielle sont radialement comprimées, la zone d'implantation artificielle étant espacée longitudinalement de l'armature,
dans laquelle dans une seconde configuration l'armature est radialement expansée, la zone d'implantation artificielle est radialement comprimée, et la zone d'implantation artificielle étant espacée longitudinalement de l'armature, et
dans laquelle dans une troisième configuration l'armature est radialement expansée, la zone d'implantation artificielle est disposée à l'intérieur de l'armature et radialement expansée, et l'armature est configurée pour être assujettie à un vaisseau.

2. Prothèse de zone d'implantation selon la revendication 1, comprenant en outre des endoancrages (410),
dans laquelle dans la troisième configuration l'armature est configurée pour être assujettie au vaisseau par l'intermédiaire des endoancrages disposés à travers l'armature et dans le vaisseau.

3. Prothèse de zone d'implantation selon la revendication 2, dans laquelle le matériau de greffon revêt au moins une partie de l'armature, et dans laquelle dans la troisième configuration les endoancrages sont configurés pour être déployés à travers le matériau de greffon.

4. Prothèse de zone d'implantation selon l'une quelconque revendication précédente, dans laquelle dans une quatrième configuration l'armature est radialement expansée, la zone d'implantation artificielle est disposée à l'intérieur de l'armature, et la zone d'implantation artificielle est radialement comprimée.

5. Prothèse de zone d'implantation selon l'une quelconque revendication précédente, dans laquelle la zone d'implantation artificielle comprend au moins une endoprothèse (246) et un matériau de greffon de zone d'implantation (252) accouplé à l'au moins une endoprothèse.

6. Prothèse de zone d'implantation selon la revendication 5, dans laquelle dans la troisième configuration la zone d'implantation artificielle est radialement expansée par l'expansion radiale de l'au moins une endoprothèse.

7. Prothèse de zone d'implantation selon la revendication 5 ou 6, dans laquelle l'au moins une endoprothèse est autoexpansible, et dans laquelle l'au moins une endoprothèse est configurée pour s'autoexpanser en libérant l'endoprothèse d'une contrainte.

8. Prothèse de zone d'implantation selon l'une quelconque revendication précédente, dans laquelle le matériau de greffon de zone d'implantation est différent du matériau de greffon.

9. Prothèse de zone d'implantation selon l'une quelconque des revendications 1 à 7, dans laquelle le matériau de greffon de zone d'implantation est une extension du matériau de greffon.

10. Prothèse de zone d'implantation selon l'une quelconque revendication précédente, dans laquelle l'armature est autoexpansible, et dans laquelle l'armature est configurée pour s'autoexpanser en libérant l'armature d'une contrainte.

11. Prothèse de zone d'implantation selon l'une quelconque revendication précédente, dans laquelle dans la troisième configuration, l'armature est assujettie au vaisseau par l'intermédiaire de l'assujettissement adhésif de l'armature et/ou du matériau de greffon fixé à l'armature au vaisseau.

12. Prothèse de zone d'implantation selon l'une quelconque revendication précédente, dans laquelle la zone d'implantation artificielle comporte en outre un accouplement externe mobile (250A, 250B) qui est configuré pour être déployé lorsque la zone d'implantation artificielle (240) est radialement expansée.

13. Système comprenant la prothèse de zone d'implantation selon la revendication 12 et une endoprothèse couverte de ramification (550A, 550B) configurée de telle sorte qu'une partie de l'endoprothèse couverte de ramification puisse être disposée à l'intérieur d'un vaisseau de ramification se ramifiant à partir du vaisseau, et dans lequel une partie de l'endoprothèse couverte de ramification est disposée à l'intérieur de l'accouplement externe mobile et vient en prise avec l'accouplement externe mobile.

14. Système selon la revendication 13, dans lequel l'endoprothèse couverte de ramification est expansible par ballonnet, et l'endoprothèse couverte de ramification est configurée pour s'expanser radialement en expansant un ballonnet disposé à l'intérieur de l'endoprothèse couverte de ramification.

15. Système selon la revendication 13 ou 14, dans lequel le matériau de greffon comporte une ouverture (266) disposée à travers celui-ci, et l'accouplement externe mobile est configuré pour être déployé de manière adjacente à l'ouverture (266) ou à travers celle-ci ; et facultativement dans lequel l'endoprothèse couverte de ramification est configurée pour être posée sur le vaisseau de ramification en posant l'endoprothèse couverte de ramification à travers l'accouplement externe mobile et l'ouverture.
